# EUROPEAN PATENT SPECIFICATION

(11) **EP 0 357 647 B1**
(45) Date of publication and mention of the grant of the patent: **28.12.1994**
(21) Application number: 88903754.5
(22) Date of filing: 28.04.1988
(51) Int. Cl.: A61N 1/32, A61N 1/34, A61N 1/36

(54) **ELECTROSTIMULATING DEVICE**
VORRICHTUNG ZUR ELEKTROSTIMULATION
DISPOSITIF D'ELECTROSTIMULATION

(30) Priority: 21.01.1988 BR 8800201
(43) Date of publication of application: 14.03.1990
(73) Proprietor: GIORDANI, Antonio, Ilson, Consalaçao, 01239-Sao Paulo, SP (BR)
(72) Inventor: GIORDANI, Antonio, Ilson, Consalaçao, 01239-Sao Paulo, SP (BR)
(74) Representative: Giambrocono, Alfonso, Dr. Ing.
(86) International application number: PCT/BR88/00005
(87) International publication number: WO 89/06554

(56) References cited:
- CH-A- 624 850
- DE-A- 3 442 760
- FR-A- 2 233 745
- FR-A- 2 598 919
- GB-A- 2 159 719
- US-A- 4 210 150

## Description

### FIELD OF INVENTION

The present invention refers to an Electrostimulating Device for applications in medical and odontological fields.

More specifically, the invention deals with a series of devices for medical, therapeutic and odontological treatments, that employ impulse-therapy or transcutaneous nervous electrostimulation, also called "TENS", as means of physiological, rational and non-invasive relaxation of specific muscular areas.

### BACKGROUND ART

Pain accompanies mankind from his birth until the end of his life. Maybe, for this reason, one type of fear, among many which exist, also accompanies man throughout his life, the fear of pain.

In odontological treatment, for example, fear of pain is almost constant, in spite of the fact that the advances in modern dentistry have been able to soothe in an extraordinary way, the unpleasant sensations experienced by the patient. Tension and anxiety still remain and constitute factors that discourage many patients from going to the dentist's, afraid of the pain they might feel. This number is quite larger than those who evade due to many other reasons added together.

The search for solutions to diminish the "stress" of odontological patients has worried dentistry. The impulsetherapy or transcutaneous nervous electrostimulation - "ENT" or "TENS", is an efficient method to achieve a physiological, rational and non-invasive relaxation, of specific muscular areas.

In 1975, Bernard Jankelson initiated studies on its applications in the odontological area, and developed a device, the "Myo-Monitor" of specific use in dentistry. Since then, many similar devices have been developed with the same purpose of relaxation and regulation of the musculature of the odonto-stomatognatic system. All of them utilize low frequency, fixed or variable (from 40 to 350 impulses per minute) to attain the proposed objectives. These devices, in spite of presenting results considered satisfactory, have several restrictions, due, mainly, to the adoption of a unique band of performance of the electrostimulating signals, the most critical being the following:
- The high frequency applied separately promotes a fast analgesic and miorelaxin effect, however of low duration after its application.
- The low frequency applied separately promotes analgesia, relaxation of longer duration, after application; however, it takes longer for the effect to initiate and, besides this, it provokes an unpleasant sensation in the patient.
- The low frequency can only be utilized up 15 to 300 pulses per minute, for above this it promotes muscular stress, which is a totally undesirable effect.

DE-A-34 42 760 discloses a device where a low-frequency modulation signal and a high-frequency carrier is generated. Other wave forms are considered in this prior art and FR-A-2 598 919 and GB-A-2 159 719 teach the use of triangular oscillators and squared-signal, amplitude-modulated oscillators for electrostimulators, respectively.

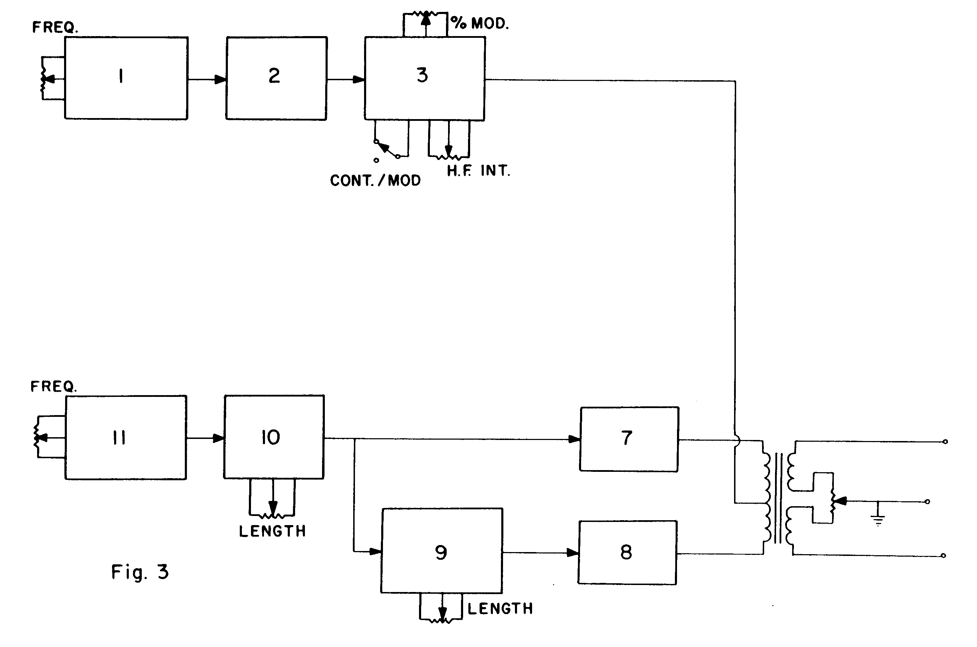

As concerns DE-A 34 42 760, it discloses an equipment which contains electronic components for generating electric signal "sets" (Pakete) (67) as well as regulating means to change and adjust the signal "set" frequency. Fig. 3 of this prior art shows the generated wave forms.

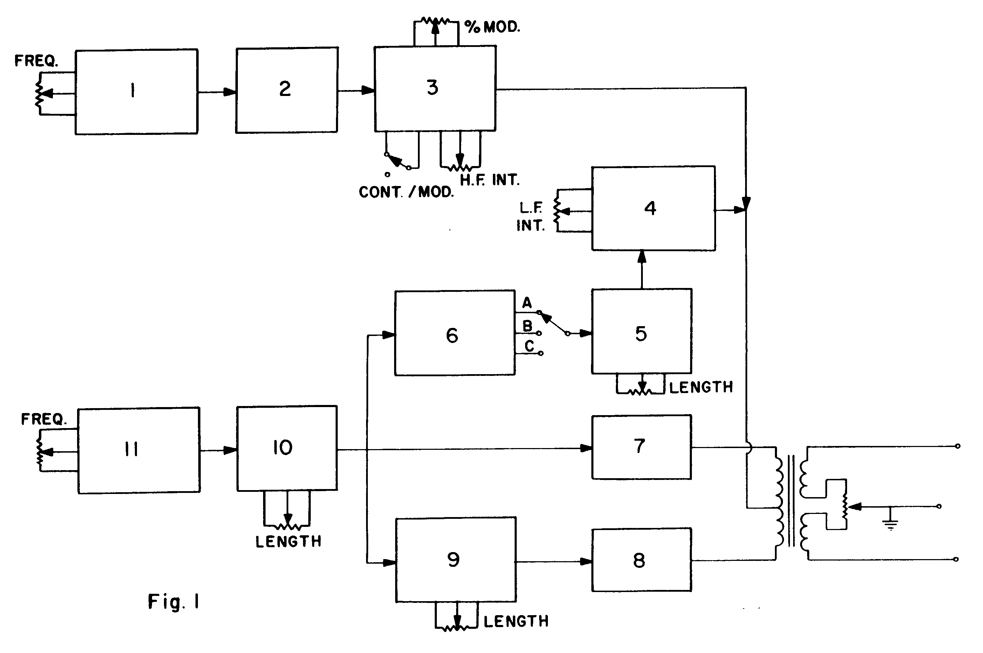

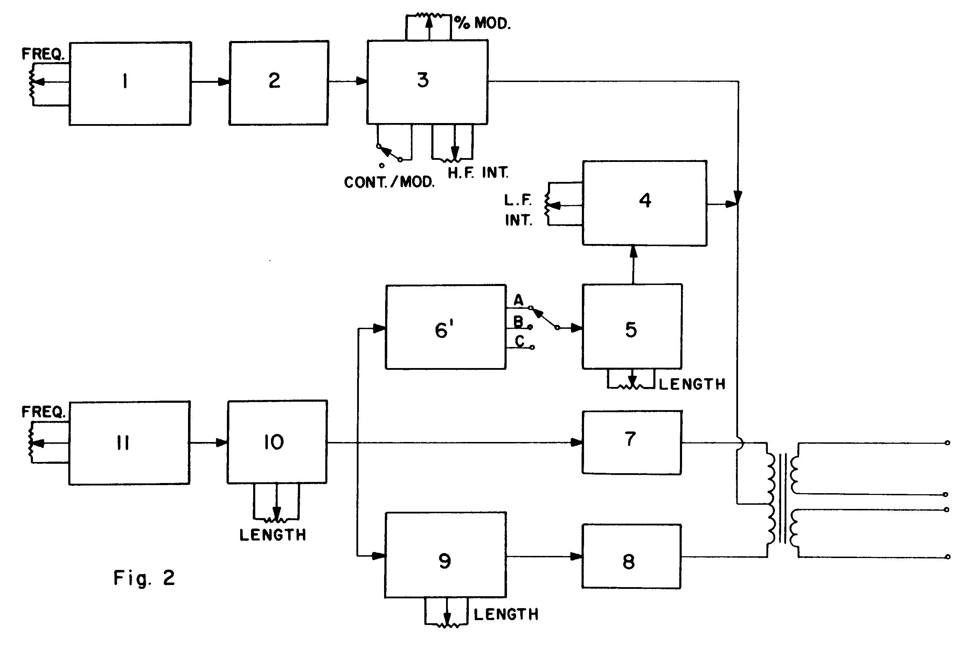

As concerns FR-A-2 598 919, it discloses a device for central electro-analgesia enabling pain suppression by means of current and tension minimal value, the influence of exceeding polarizations on the electrodes in prolonged surgeries and also preventing the patient from suffering unpleasant sensation at the electrode application sites due to the same reasons (page 4, lines 20-27). The device is represented in block format in fig. 1 and in more constructive circuit format in Fig. 2.

As concerns GB-A-2 159 719, it discloses an electronic glaucoma treatment apparatus which uses a transcutaneous electronic wave to suppress pain and reduce intra-ocular pressure. This known apparatus applies an electronic current wave comprising relatively high-frequency pulses with a low-frequency amplitude modulation applied between the first and second electrodes. The apparatus is represented in block format in Fig. 1 and the wave forms are shown in Figs. 2A-D. The frequency of the high-frequency electric wave ranges from 12 to 20 kHz and the low-frequency modulation ranges from 8 to 20 Hz, with the referred wave not exceeding 4 mA. The amplitude modulation is non-symmetrical.

The analysis of the above prior art reveals the following:
- The three documents disclose electronic equipments generating electric waves to suppress pain in patients;
- DE-A-34 42 760 and GB-A-2 159 719 emphasize the use of high frequency and low frequency and have the shortcomings illustrated in the present patent application (from page 1, line 26 to page 2, line 16);
- none of the cited documents teaches or suggests the use of both high and low frequency modulation as provided for in the present application.

CH-A-624 850 describes an electrostimulating device suitable for producing an electric current in order to act on body muscles so as to obtain muscle contraction without causing pains to the patient.

In order to achieve this, the known device combines three different sinusoidal current having different frequencies, i.e. an average physiological frequency, a low frequency and a very low frequency; the latter current is used as modulating current in combination with the other current signals. In particular, the prior document discloses to combine in a first stage, the average frequency current and the the very low frequency current (or modulating current) and the latter (shifted by 180°) and the low frequency current; in a second stage, in order to have a painless stimulation of the patient body, the known device discloses to combine (in particular, to add) the above-mentioned current combinations.

The last-mentioned combination is obtained by feeding the combined signals to adding circuits which add the previous combined currents through two transformers connected in series which, in turn, are connected to electrodes to get in contact with the patient skin. Each pair of transformers is connected to one electrode.

The developed electrostimulating device presents technical innovations, which resulted from experiences and research carried out with other miostimulating devices, which indicated that the incorporation of high frequency, modulated to high and low frequencies, isolated or superposed, improved appreciably the efficiency of the "TENS" results, in the treatment of the symptomatology of pain and in the musculature relaxation.

Thus, the following may be pointed out as the main innovations of the electrostimulating device:
- Incorporation of the modulation of the low frequency pulses, in those of high frequency, in frequency as well as in phase. Under these conditions, the characteristics of the pulse length are kept stable and adjustments are allowed regardless of intensity;
- Introduction of the modulation of the high frequency signal by another low frequency signal (in the range of 0,5 to 8,333 Hz), which wave may be triangular or sinusoidal. Internally, the modulation may he adjusted between 0 to 100%. Practical experiments indicated a modulation factor of 50% as being of good efficiency in the impulse therapy applications, due to the remnant effect of the continuous low frequency signal; and
- Combined utilization of the resources described above, which allows the therapeutic application of the three signals simultaneously.

Such innovations increase the field of application of the device, due to the combination of frequencies which it permits. Resulting from this, devices have been projected for odontological, physiotherapeutic and therapeutic applications, constituting a series of miostimulating devices.

For the obtention of the outlet functions, the electrostimulator consists of two generators of base time and associated circuits. One of the generators is destined to the generation of high and low frequency signals and the other, to the generation of the signal which constitutes the modulation.

The characteristic of the form of the outlet wave is of the squared alternated type or approximately squared in both alternances, or still, squared in one of the alternances (positive or negative) with or without exponential in the other. Other possible forms of waves are: triangualar, sinusoidal, double-phase or monophase type.

Each stimulus must have the pulse length adjustable from 60 to 20.000 microseconds and current limitation of a maximum of 50 milliamperes, and the signal that composes each stimulus may be of the unique type or the "burst" type.

The electrostimulating device object of this invention, will be better understood in connection with the annexed drawings, which schematically represent:
Fig. 1 - diagram of blocks representing the functional structure of the electrostimulating device for odontological applications, representing the basic units which constitute it.
Figure 2 - diagram of blocks representing the functional structures of a different form of the electrostimulating device, applied to other medical and odontological fields.
Figures 3, 4 - diagrams of block representing known functional structures which can be obtained from the structure of fig. 1;
Figure 5 - scheme of the basic circuit of the triangular signal generator unit, amplifying unit, modulation control unit and measuring unit.
Figure 6 - scheme of the basic circuit of the squared signal generator unit, 1^{st} stage signal conformator, unit, dividing unit, 2^{nd} stage signal conformator unit, 3^{rd} stage signal conformator unit, low frequency signal controller unit, final amplification unit and outlet transformer unit.

In Figure 1 where the constitution of the device is shown for odontological application, including the basic units of all the others shown in figures 2, 3 and 4, it may be observed that the electrostimulating device includes a triangular signal generator (1), followed by the amplifier (2), which is connected to the modulation controller (3). A squared signal generator (11) which may also generate other wave forms, according to therapeutic needs, is connected to a 1^{st} stage signal conformator (10), which, in its turn, is parallely connected to a frequency divider (6), to a final amplifier (7) and to another 2^{nd} stage signal conformator (9), and another final amplifier (8) follows the latter. The referred divider (6) is still connected through a key of three positions (A, B, C) to a 3^{rd} stage signal conformator (5) and this one to a low frequency signal controller (4). This device presents the following characteristic's:
- Main applications: relaxation, and unprogramming of the chewing and facial muscles, mandibular monitoring and other electrotherapy applications in the facial region.
- Technical functions: continuous high frequency (100 Hz), modulated high frequency (0,666 Hz modulation), low frequency (40 ppm, 100 ppm, 300 ppm), continuous high frequency juxtaposed by the low frequency, modulated high frequency juxtaposed by the low frequency.
- Control: high frequency intensity, low frequency intensity, equilibrium, low frequency selector, continuous/modulated high frequency selector, turn-on/turn-off.
- Outlets: two active poles, right and left, a diffusing pole.

In figure 2, the diagram of blocks of a variant of the device is shown, which, as an example of the others, presents a basic structure similar to that of figure 1, the main difference being the substitution of the divider (6) by a divider (6') having characteristics different from those of figure 1. The referred device has as main characteristics the following:
- Main applications: analgesia, muscular stimulation, cicatricial process activation, general electrotherapy.
- Technical functions: continuous high frequency (100 Hz), modulated high frequency (0,666 Hz modulation), low frequency (80 ppm, 160 ppm, 300 ppm), continuous high frequency juxtaposed by the low frequency, modulated high frequency juxtaposed by the low frequency.
- Controls: high frequency intensity, low frequency intensity, continuous/modulated high frequency selector, turn-on/turn-off low frequency selector.
- Outlets: two or more positive poles, two or more negative poles.

Figure 3, shows the diagram of a device operating in a known way (and therefore not subject-matter of the present invention) and whose structure is generated by the suppression of the low frequency controller (4), of the 3^{rd} stage signal conformator (5) and of the divider (6), being still possible the suppression of the 1^{st} and 2^{nd} stage signal conformators (10; 9).

This known operating device presents the following characteristics:
- Main applications: relaxation, analgesia, lymphatic drainage, cellular revitalization.
- Technical functions: continuous high frequency (100 Hz), modulated high frequency (modulation with 0,666 Hz).
- Controls: intensity, equilibrium, continuous/modulated, turn-on/turn-off high frequency selector.
- Outlets: two active poles, right and left, a diffusing pole.

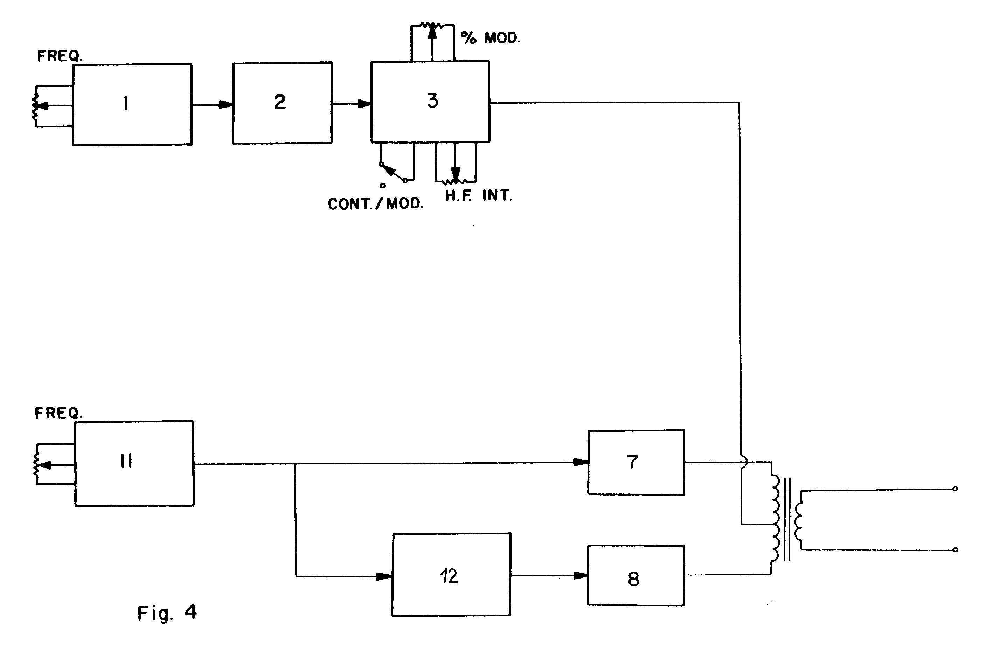

In figure 4, it is observed the diagram of the functional structure of another device, operating in a known way (and therefore not subject matter of the present invention) and having units which are similar to those of the device in figure 3, however, being here suppressed the 1^{st} and 2^{nd} stage signal conformators and an inverter (12) is introduced in their place and connected to the final amplifier (8). Its main characteristics are:
- Applications: analgesia, relaxation, stimulation.
- Technical functions: continuous high frequency (100 Hz), modulated high frequency (0,666 Hz modulation).
- Controls: continuous/modulated high frequency selector.
- Outlets: two poles, a positive and a negative one.

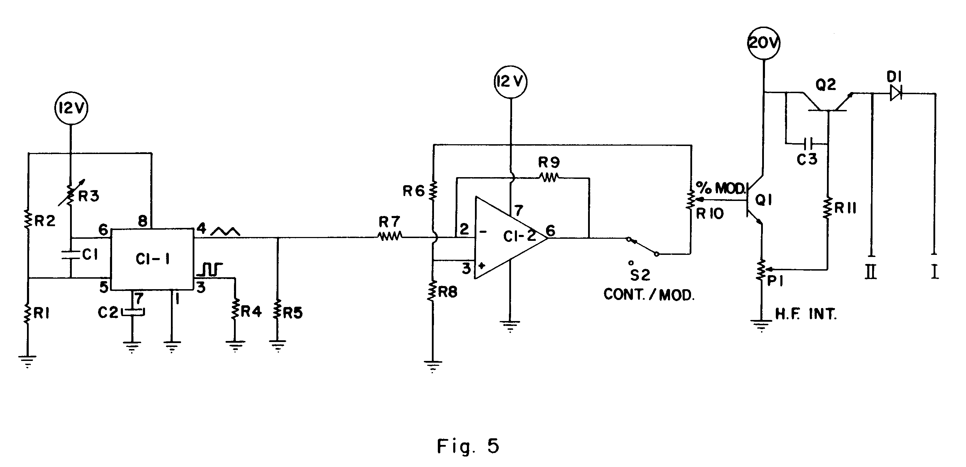

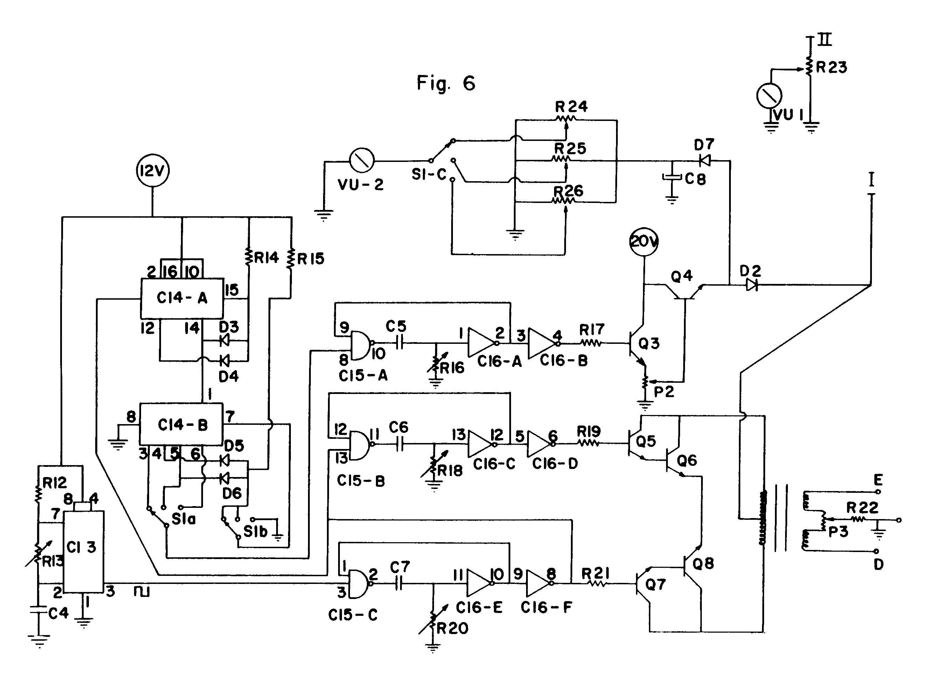

Figures 5 and 6 associated, form the general basic circuit of the electrostimulating device of the invention, according to figure 1.

In this way, it may be observed by figure 5 that the developed device includes a generating unit of triangular signal, in which the high frequency modulation is of the "modulation by amplitude" type, with modulation frequency of 0,666 Hz, of the triangular or sinusoidal type. This unit contains an oscillator which consists of a CI-1 circuit and other circuit supports, which is controlled by tension and with triangular (pin 4) and squared (pin 3) outlet wave. The adjustment of the frequency is achieved by R3 and C2. From the CI-1 outlet, only the triangular signal (pin 4) is utilized for the feeding of the operational amplifier CI-3 of the inverter type with an approximate tenfold gain. The referred operational amplifier CI-2, varies the base polarity of the Q1 transistor of the modulation controller, by a variable resistor R10. In the controller, the low frequency is controlled by means of variations in P1, R11 and Q2, associated to variations in Q1. These variations are cadenced with the above-mentioned frequency of the oscillator, thus obtaining the triangular or sinusoidal modulation of the high frequency signal, the percentual control of the modulation being obtained by the variable resistor R10. A key S2 acts to impose or not the modulation, and when it is in the turned off position, the base of the Q1 transistor becomes positively polarized and it passes to conduct the current, allowing, in this form, to adjust the high frequency intensity by means of P1.

The visual control of the outlet signal (relative control) in Q2 and Q4 is carried out by instruments of the "VU" type, and the reading of the bottom of the scale is adjusted at high frequency by R23 and at low frequency by R24, R25 and R26, being selected by frequency in the S1-C key, the low frequency pulses being amortized by D7 and C8 and, consequently, the visualization of the reading of the measuring instrument is greatly improved. This measurement may also be obtained with leds.

In figure 6, one may observe the squared signal generator, responsible for the high frequency stimuli, which is obtained by an oscillator composed by the integrated circuit CI-3.

This circuit, in the represented configuration, supplies an outlet signal in pin 3 in the form of a squared wave, resistors R12, R13 and capacitor C4 forming the time constant that determine the oscillation frequency. With resistor R13, which is of the variable type, the generator may be adjusted in the desired frequency.

The signal emitted by CI-3 is then sent to the 1^{st} stage signal conformator, which consists of a monostable circuit, formed by a logical part of the "NAND" type - CI5-C circuit - and by two inverter circuits of the "Schmitt Trigger" type - CI6-E and CI6-F, being that in this unit, resistor R20 and capacitor C7 form the time constant that adjust the length of the pulse to be obtained at the outlet of transformer T1. The referred monostable circuit is sensitive to the descent limit of the squared wave signal.

Pin 8 of the CI6-F circuit feeds a final amplifier (7) with rectangular pulses of length defined by the referred monostable circuit and in the established frequency, as well as the second stage signal conformator (9) and the frequency divider block (6).

The signal of the conformator stage (10) feeds, as stated above, the 2^{nd} conformator stage (9) that consists of circuits CI5-B, CI6-C and CI6-D and which outlet feeds, in its turn, a second final amplifier (8).

The referred final amplifiers (7; 8) are transistorized and in "Darlington" configuration (Q7, Q8, Q6 and Q5), being each one connected to one of the prime windings of the outlet transformer.

The outlet transformer is fed in its center by a continuous tension, which when added to the two sequential signals of the signal conformator stages, result in a reversion of the magnetic flow in the transformer, producing by induction in the secondary coils, an alternated signal of positive and negative semicycle lengths proportional to the lengths adjusted in the two monostable circuits. The variation of intensity of the outlet signal is obtained by the variation of the tension level that feeds the center of the transformer. This effect is achieved by the variation in P1 and in the base polarization of Q2.

The low frequency stimuli are obtained from the outlet of the 1^{st} stage signal conformator, circuits CI4-A and CI4-B forming the frequency divider circuits.

In reality, these circuits are synchronous meters of the binary type, the first circuit (CI4-A) is configurated to divide by 10 and in the second (CI4-B) circuit the subdivisions by 2,6 and 15 are selected, which permits to obtain the desired frequencies from the original frequency.

These pulses obtained from the divider circuit are selected by key S1-a and sent to the 3^{rd} stage signal conformator, which constitution is identical to the previous ones (1^{st} and 2^{nd}) and formed by circuits CI5-A, CI6-A and CI6-B. in this conformating stage, the length of the pulse is adjusted by resistor R16, being obtained the double of time of the length of the pulse when compared to the 1^{st} and 2^{nd} signal corformator stages.

The outlet of the 3^{rd} conformating stage feeds transistor Q3 of the low frequency signal controller (4), which conducts only the low frequency pulses. This transistor Q3 and potentiometer P2 form the potentiometer which commands the polarization of Q4 which, by its turn, controls the tension level that feeds the circuit of the primary winding of the outlet transformer. As the low frequency pulses are obtained from the high frequency oscillator by means of potentiometers, they are always synchronized with the high frequency.

The developed device also has diodes D1 and D2 to block eventual inverse polarizations of transistors Q2 and Q4.

Thus, the high frequency outlet juxtaposed by low frequency is obtained, selected and with independent intensity controllers, whereas at the outlet of the device, an outlet transformer is placed, of the elevating-tension type, with two independent secondary windings and interconnected by the equilibrium potentionmeter P3, which carries out the balancing of the stimuli applied to electrodes E and D, in contraposition to eventual differences of resistance of contact with the human skin.

The above description, as previously mentioned, is related to the basic electrostimulating device; shown in the diagram of blocks of figure 1, which permits variations in the form of outlet signal measurement, in the form of feeding or still in the outlet stimuli of the alternated type with a semicycle in the form of squared wave and another in exponential form. Time controllers and many other resources may also be introduced in the device.

The functions presented at the outlet of the electrostimulating device according to the invention are:
- Continuous High Frequency: it is a signal with a double-phase or monophase wave form as has already been described, intensity adjustable from 0 to 100% - with a frequency in the order of 100 Hz, which can vary from 50 to 5.000 Hz.
- Low Frequency: it is a double-phase or monophase signal as already described, frequency adjustable in a range from 0,333 to 10 Hz (internally) and may be selected by the user in the frequencies of 0,666 Hz (40 ppm), 1,666...Hz (100 ppm) and 5 Hz (300 ppm), such selected adjustments being able to change according to the best utilization convenience.

The duration of the low frequency pulse is adjustable according to clinical needs.

The intensity is in the case continuously adjustable from 0 to 100%.
- High Frequency with Superposed Low Frequency: consists of a signal as described for continuous high frequency and another as described for low frequency, existing in this case adjustments which are independent of intensity, where the low frequency signal should juxtapose the high frequency signal, and such juxtaposition, must occur synchronized in phase and frequency. This juxtaposition is achieved by the High Frequency modulation, where, in a repetitive and ajustable periodicity, a high frequency alternance has its intensity increased.
- Conjugation of the Continuous High Frequency, Low Frequency and Modulated High Frequency: according to the characterization of the name itself, it consists of a combination of stimuli where the modulated high frequency is juxtaposed by the low frequency, whereas only the juxtaposition of the High Frequency and the Low Frequency is made in a synchronized form, which does not occur with the modulation signal and the low frequency signal, even if both have the same frequency.

The constituent devices of the miostimulating series which the invention refers to utilize the basic circuits projected for the odontological application device. This device, as well as the others may be commercialized in more than one model, that is "standard" or "Luxe", the models differing in their finishing, by complementary electronic circuits, such as time programmer, current meter, automatic turn-off, etc.

All the devices consist of a feeding source for utilization in the commercial net of electric energy, being possible the fabrication for use with batteries or electric cells.

## Claims

1. An electrostimulating device comprising: a first signal generator (1) for generating a first signal; a unit (Q1, P1, R11, Q2, C3, D1) receiving said first signal to generate a modulated signal which is conveyed to a transformer connected to electrodes (E, D); a second signal generator (11) for generating a second signal for said transformer,
characterized in that
a) the said unit (Q1, P1, R11, Q2, C3, D1) forms part of a modulation controller (3) with variable modulation factor and output signal intensity (through R10, S2), whose output signal is connected to the center point of the transformer primary winding;
b) said second signal generator (11) comprises a frequency adjustable square wave generator (11) with an output connected to a first stage signal conformator (10) - adjusting the length of the square wave signal impulses - which in turn has an output connected in parallel to a frequency divider (6), a first amplifier (7) connected to the transformer at one end of the primary winding, and also to a second stage signal conformator (9);
c) the output of the second stage signal conformator (9) is connected to a second amplifier (8) having an output connected to the transformer to the other end of the primary winding;
d) the output of the frequency divider (6) is connected to a third stage signal conformator (5) and the latter to a low-frequency signal controller (4) conducting only pulses of a predetermined frequency, and the output signal of said controller (4) is also connected to the center point of the transformer primary winding;
e) said first signal generator (1) generates a triangular or sinusoïdal waveform.

2. A device as in claim 1, wherein the output transformer has a plurality of balanced, interconnected output windings connected to corresponding electrodes (E, D).

3. A device as in claim 1, wherein the triangular wave generator (1) is connected to an amplifier including an inverting type operational amplifier (CI-2) having a predetermined gain with an output thereof coupled to said modulation controller (3).

4. A device as in claim 1, wherein said square wave generator includes an adjustable oscillator (CI-3).

5. A device as in claim 1, wherein each of said signal conformators includes an adjustable monostable circuit (CI5-C, CI6-E, CI6-F etc.)

6. A device as in claim 1, wherein said amplifier (7, 8) includes a Darlington-type solid state amplifier (Q7, Q8, Q6 and Q5);

## Patentansprüche

1. Elektrostimuliervorrichtung mit: einem ersten Signalgenerator (1) zum Erzeugen eines ersten Signals; mit einer Einheit (Q1, P1, R11, Q2, C3, D1), die das erste Signal aufnimmt, um ein moduliertes Signal zu erzeugen, das an einen Transformator übertragen wird, der an Elektroden (E, D) angeschlossen ist; mit einem zweiten Signalgenerator (11) zum Erzeugen eines zweiten Signals für den Transformator,
**dadurch gekennzeichnet,**
(a) daß die Einheit (Q1, P1, R11, Q2, C3, D1) Teil einer Modulationssteuerung (3) mit einem variablen Modulationsfaktor und einer Ausgangssignalintensität (über R10, S2) ist, deren Ausgangssignal an den Mittelabgriff der Transformator-Primärwicklung gelegt ist;
(b) daß der zweite Signalgenerator (11) einen frequenzeinstellbaren Rechteckwellen-Generator (11) aufweist, von dem ein Ausgang an einen ersten Signalgleichmacher (10) angeschlossen ist, der die Länge der Rechteckwellen-Signalimpulse einstellt, und der mit einem Ausgang parallel an einen Frequenzteiler (6), einen ersten Verstärker (7), der an ein Ende der Transformator-Primärwicklung angeschlossen ist, und außerdem an einen zweiten Signalgleichmacher (9) angeschlosse ist;
(c) daß der Ausgang des zweiten Signalgleichmachers (9) an einen zweiten Verstärker (8) angeschlossen ist, der mit einem Ausgang am anderen Ende der Transformator-Primärwicklung liegt;
(d) daß der Ausgang des Frequenzteilers (6) an einen dritten Signalgleichmacher (5) angeschlossen ist, der wiederum an eine Niederfrequenz-Signalsteuerung (4) angeschlossen ist, die lediglich Impulse einer vorgegebenen Frequenz verarbeitet, und daß die Ausgangssignale der Steuerung (4) auch an den Mittelabgriff der Transformator-Primärwicklung gelegt werden; und
(e) daß der erste Signalgenerator eine Dreieckschwingung oder eine Sinusschwingung erzeugt.

2. Vorrichtung nach Anspruch 1, wobei der Ausgangstransformator eine Anzahl von abgeglichenen, untereinander verbundenen Ausgangswicklungen aufweist, die an entsprechende Elektroden (E, D) angeschlossen sind.

3. Vorrichtung nach Anspruch 1, wobei der Dreieckschwingungsgenerator (1) an einen Verstärker mit einem invertierenden Operationsverstärker (CI-2) angeschlossen ist, der eine vorgegebene Verstärkung besitzt und dessen Ausgang an der Modulationssteuerung (3) liegt.

4. Vorrichtung nach Anspruch 1, wobei der Rechteckschwingungsgenerator einen einstellbaren Oszillator (CI-3) aufweist.

5. Vorrichtung nach Anspruch 1, wobei jeder der Signalgleichmacher eine einstellbare monostabile Schaltung (CI5-C, CI6-E, CI6-F, etc.) aufweist.

6. Vorrichtung nach Anspruch 1, wobei der Verstärker (7, 8) einen Darlington-Festkörperverstärker (Q7, Q8, Q6 und Q5) aufweist.

## Revendications

1. Dispositif d'électrostimulation comprenant : un premier générateur de signal (1) pour générer un premier signal ; une unité (Q1, P1, R11, Q2, C3, D1) recevant ledit premier signal pour produire un signal modulé qui est envoyé sur un transformateur relié à des électrodes (E, D) ; un second générateur de signal (11) pour produire un second signal pour ledit transformateur, caractérisé en ce que :
a) ladite unité (Q1, P1, R11, Q2, C3, D1) fait partie d'une commande de modulation (3) dont le facteur de modulation et l'intensité du signal de sortie sont variables (par R10, S2), ledit signal de sortie étant relié au point milieu de l'enroulement primaire du transformateur ;
b) ledit second générateur de signal (11) comprend un générateur d'onde carrée à fréquence ajustable (11) dont la sortie est reliée à un premier étage adaptateur de signal (10) - pour ajuster la longueur des impulsions du signal carré - dont la sortie, à son tour, est raccordée en parallèle sur un diviseur de fréquence (6), sur un premier amplificateur (7) relié à l'une des extrémités de l'enroulement primaire du transformateur, et également sur un second étage adaptateur de signal (9) ;
c) la sortie du second étage adaptateur de signal (9) est reliée à un second amplificateur (8) dont la sortie est reliée à l'autre extrémité de l'enroulement primaire du transformateur ;
d) la sortie du diviseur de fréquence (6) est reliée à un troisième étage adaptateur de signal (5), et ce dernier à une commande de signal basse fréquence (4) qui ne transmet que les impulsions ayant une fréquence prédéterminée, le signal de sortie de ladite commande (4) étant également relié au point milieu de l'enroulement primaire du transformateur ;
e) ledit premier générateur de signal (1) délivre une onde de forme triangulaire ou sinusoïdale.

2. Dispositif selon la revendication 1, caractérisé en ce que le transformateur de sortie comporte plusieurs enroulements secondaires en phase interconnectés, raccordés aux électrodes correspondantes (E, D).

3. Dispositif selon la revendication 1, caractérisé en ce que le générateur d'onde triangulaire (1) est relié à un amplificateur comportant un amplificateur opérationnel de type inverseur (CI-2) ayant un gain prédéterminé et dont la sortie est couplée à ladite commande de modulation (3).

4. Dispositif selon la revendication 1, caractérisé en ce que ledit générateur d'onde carrée comporte un oscillateur ajustable (CI-3).

5. Dispositif selon la revendication 1, caractérisé en ce que chacun desdits adaptateurs de signal comporte un circuit monostable ajustable (CI5-C, CI6-E, CI6-F, etc.).

6. Dispositif selon la revendication 1, caractérisé en ce que ledit amplificateur (7, 8) comporte un amplificateur à semi-conducteurs de type Darlington (Q7, Q8, Q6 et Q5).
